# EUROPEAN PATENT SPECIFICATION

(11) **EP 3 934 556 B1**
(45) Date of publication and mention of the grant of the patent: **07.01.2026**
(21) Application number: 20766885.6
(22) Date of filing: 04.03.2020
(51) Int. Cl.: A61B 34/00, A61B 18/12, A61B 50/13, A61B 17/34, A61M 13/00, A61B 90/00, A61B 50/22

(54) **AUXILIARY FUNCTION CONTROL APPARATUS FOR MEDICAL DEVICES, AND RELATED SYSTEMS**
HILFSFUNKTIONSSTEUERGERÄT FÜR MEDIZINISCHE VORRICHTUNGEN SOWIE ZUGEHÖRIGE SYSTEME
APPAREIL DE COMMANDE À FONCTION AUXILIAIRE POUR DISPOSITIFS MÉDICAUX, ET SYSTÈMES

(30) Priority: 05.03.2019 US 201962813977 P
(43) Date of publication of application: 12.01.2022
(73) Proprietor: Intuitive Surgical Operations, Inc., Sunnyvale CA 94086-5301 (US)
(72) Inventor: ETTINGER, Gary, Sunnyvale, California 94086 (US); BLANCHARD, Russell, Sunnyvale, California 94086 (US); GOTSILL, Craig, Sunnyvale, California 94086 (US); OSHATZ, Daryl, Sunnyvale, California 94086 (US); ZABINSKI, John W., Sunnyvale, California 94086 (US)
(74) Representative: MacDougall, Alan John Shaw
(86) International application number: PCT/US2020/020917
(87) International publication number: WO 2020/180944

(56) References cited:
- WO-A1-2008/053485
- WO-A1-2017/066741
- US-A1- 2008 255 902
- US-A1- 2010 098 425
- US-A1- 2013 223 684
- US-A1- 2015 223 865
- US-A1- 2015 257 814
- US-A1- 2016 192 998
- US-B1- 6 251 113
- US-B2- 7 793 222

## Description

### CROSS-REFERENCE TO RELATED APPLICATIONS

This application claims priority to U.S. Provisional Application No. 62/813,977, filed March 5, 2019.

### TECHNICAL FIELD

Aspects of the present disclosure relate to an auxiliary function control apparatus for connection to and control over medical devices during a surgical procedure, such as for example a minimally-invasive surgical procedure employing computer-assisted technology. Further aspects of the present disclosure relate to user interfaces for auxiliary function control apparatuses.

### INTRODUCTION

Minimally invasive surgery seeks to minimize patient trauma by introducing therapeutic, diagnostic, and imaging instruments through small incisions or natural orifices. One group of such instruments are manually operated. Another group of such instruments are teleoperated by using a computer-assisted surgical system (a telesurgical system), in which a surgeon operates an input control unit to remotely control one or more instruments operated by a manipulator unit to which the one or more instruments are coupled.

Whether operated manually or via a telesurgical system, many instruments are coupled to auxiliary function units that support the instruments' clinical purpose. For example, electrosurgical instruments are coupled to electrosurgical generator units (ESUs) that provide mono- and bipolar energy to the instrument as required. Likewise, suction/irrigation devices that may be used during various surgeries require operable coupling to corresponding irrigation fluid and vacuum sources. Similarly, endoscope instruments require supporting imaging and illumination equipment, which is typically provided in one or more separate auxiliary function units. Thus, various auxiliary function equipment is typically used during minimally invasive surgery to support medical devices used at a remote surgical site in performing functions such as insufflation supply, cautery smoke evacuation, ultrasonic energy generation, etc. As used herein, medical devices include both instruments used for manipulating tissue and sensing an environment (e.g., imaging, pressure, oxygen etc.) at a remote surgical site, as well as devices used to supply suction, irrigation, light, and other types of flux proximate a remote surgical site.

As a result of the many auxiliary function units that are used to support modern surgery, and the various connections (electrical cables, tubes, etc.) between the auxiliary function units and the corresponding medical devices, the operating room becomes a complex and crowded environment during surgery. Each individual auxiliary function unit may be placed in a different location, and each unit's user interface to control its functions may be different and challenging to easily and quickly access. This problem is especially true because it is important to preserve medical device sterility for surgeons who use manually-operated medical devices, and access to auxiliary function units may be difficult for surgeons who use telesurgical systems and are physically remote from the patient. Accordingly, operating room staff are often relied on to operate the auxiliary function units. Such personnel may need to move to various locations around the patient and work around various transmission lines (e.g., electrical cables, fluid supply tubes, etc.) in order to reach the various auxiliary function unit user interfaces.

Yet another user interface problem occurs when positioning various auxiliary function units near each other, because the physical characteristics of one auxiliary function unit may cause an interference with one or more other units. For example, if a first auxiliary function unit is placed above a second auxiliary function unit in a single rack (e.g., shelving unit), transmission lines from the first auxiliary function unit may fall in front of the second auxiliary function unit's user interface or cable connectors, thereby potentially making access to the second auxiliary function unit difficult and/or potentially obscuring a display on the second auxiliary function unit. Such a "waterfall" of transmission lines from a plurality of auxiliary function units may further limit access to and visibility of any auxiliary function units positioned underneath others.

Therefore, a need exists to simplify operating room management and use of the variety of separate auxiliary function units used during surgery. A need also exists to improve user interfaces of the various auxiliary function units that are used to enhance medical device functions and perform other surgically relevant tasks.

WO2008/053485 describes an apparatus for treatment of a body of a patient includes one or more functional modules. Each functional module includes at least one connector for connection to a respective functional element that is applied to the body of the patient, respective user controls, for controlling operation of the respective functional element, and a respective communication interface, which is configured to receive signals for remote control of the respective functional element. A central control unit includes a user interface for receiving user inputs for controlling the one or more functional modules. Control circuitry is operative to deactivate the respective user controls of the functional modules while the functional modules are under control by the central control unit, and to activate the respective user controls upon occurrence of a predetermined condition.

US 2015/0257814 describes a flux supply unit for supplying a flux to a plurality of surgical instruments may include a plurality of connectors and a user control interface. The plurality of connectors may be configured to supply flux to the surgical instruments when the surgical instruments are operationally coupled to respective connectors. The user control interface may include a continuous display screen including a plurality of graphical display screen sections on the same display screen that display controls for surgical instruments operationally coupled to the plurality of connectors. The plurality of graphical display screen sections may display controls for different types of surgical instruments on the same continuous display screen. The plurality of graphical display screen sections may be arranged relative to the plurality of connectors to visually couple respective graphical display screen sections and connectors to indicate respective associations of the graphical display screen sections with the connectors.

US 2015/0223865 describes a system and method for transmitting electrosurgical energy from a generator to an electrosurgical instrument are provided. The electrosurgical system includes a generator adapted to generate electro surgical energy for treating tissue. The generator includes one or more active output terminals which supply energy to the tissue. The active output terminals are operatively connected to one or more active leads. The generator also includes one or more return output terminals which returns energy from the tissue. The return output terminals are operatively connected to at least one return lead. The system also includes an electro surgical instrument operatively connected to the one or more active leads and one or more return electrodes operatively connected to one or more return leads. The system further includes an electrosurgical cable including one or more active leads and one or more return leads. The one or more active leads and one or more return leads are wound in a double helix fashion such that the electrical field along the cable is mitigated along the length thereof.

US 6,251,113 describes a system for controlling a plurality of ophthalmic microsurgical instruments connected thereto. The microsurgical instruments are for use by a user such as a surgeon in performing ophthalmic surgical procedures. The system includes a data communications bus and a user interface connected to the data communications bus. The user interface provides information to the user and receives information from the user which is representative of operating parameters of the microsurgical instruments. The system also includes surgical modules connected to and controlling the microsurgical instruments as a function of at least one of the operating parameters. The surgical modules are also connected to the data communications bus. The data communications bus provides communication of data representative of the operating parameters between the user interface and the surgical modules. Other features are also disclosed including a main control, an endo-illuminator system, a phacoemulsification handpiece, surgical scissors, a vitrectomy cutter, a surgical foot control, a remote control, a cart.

### SUMMARY

The present invention is defined by appended claim 1. Further, embodiments are disclosed in the dependent claims. Exemplary embodiments of the present disclosure may demonstrate one or more of the above-mentioned desirable features. Other features and/or advantages may become apparent from the description that follows.

The present invention relates to an apparatus comprising a cart; a first auxiliary function unit removably housed by the cart, the first auxiliary function unit comprising one or more first connector interfaces, the one or more first connector interfaces configured to be operably connected to one or more medical devices via mating engagement with one or more first transmission lines; a second auxiliary function unit removably housed by the cart, the second auxiliary function unit comprising one or more second connector interfaces, the one or more second connector interfaces configured to be operably connected to one or more medical devices via mating engagement with one or more second transmission lines; and a user interface overlay panel coupled to the cart and overlying at least a portion of each of the first auxiliary function unit and the second auxiliary function unit, wherein: the user interface overlay panel comprises: one or more first port openings overlaying the one or more first connector interfaces and configured to receive one or more first transmission line connectors of the one or more first transmission lines, one or more second port openings overlaying the one or more second connector interfaces and configured to receive one or more second transmission line connectors, and a user interface control portion operably coupled to each of the first auxiliary function unit and second auxiliary function unit to alter control settings of each of the first auxiliary function unit and second auxiliary function unit in response to input at the user interface control portion, wherein the user interface control portion comprises: a first user interface control area aligned with the one or more first connector interfaces, the first user interface control area configured to receive input to control the first auxiliary function unit, and a second user interface control area aligned with the one or more second connector interfaces, the second user interface control area configured to receive input to control the second auxiliary function unit.

Additional objects, features, and/or advantages will be set forth in part in the description which follows, and in part will be obvious from the description, or may be learned by practice of the present disclosure and/or claims. At least some of these objects and advantages may be realized and attained by the elements and combinations particularly pointed out in the appended claims.

It is to be understood that both the foregoing general description and the following detailed description are exemplary and explanatory only and are not restrictive of the claims; rather the claims should be entitled to their full breadth of scope, including equivalents.

### BRIEF DESCRIPTION OF THE DRAWINGS

The present disclosure can be understood from the following detailed description, either alone or together with the accompanying drawings. The drawings are included to provide a further understanding of the present disclosure and are incorporated in and constitute a part of this specification. The drawings illustrate one or more exemplary embodiments of the present teachings and together with the description serve to explain certain principles and operation. In the drawings:
FIG. 1 is a perspective, diagrammatic view of an auxiliary function control apparatus according to an exemplary embodiment of the present disclosure.
FIG. 2 is a front view of the apparatus of FIG. 1.
FIG. 3 is a front, diagrammatic view of another auxiliary function control apparatus according to an exemplary embodiment of the present disclosure.
FIG. 4 is a front, diagrammatic view of another auxiliary function control apparatus according to an exemplary embodiment of the present disclosure.
FIG. 5 is a front, diagrammatic view of another auxiliary function control apparatus according to an exemplary embodiment of the present disclosure.
FIG. 6 is a perspective, diagrammatic view of another auxiliary function control apparatus according to an exemplary embodiment of the present disclosure.
FIG. 7 is a front view of the apparatus of FIG. 6.
FIG. 8 is a front, diagrammatic view of another auxiliary function control apparatus according to an exemplary embodiment of the present disclosure.
FIG. 9 is a front, diagrammatic view of another auxiliary function control apparatus according to an exemplary embodiment of the present disclosure.
FIG. 10 is a front, diagrammatic view of another auxiliary function control apparatus according to an exemplary embodiment of the present disclosure.
FIG. 11 is a perspective, diagrammatic view of another auxiliary function control apparatus according to an exemplary embodiment of the present invention.
FIG. 12 is a front view of the apparatus of FIG. 11.
FIG. 13 is a front view, diagrammatic of the auxiliary function control apparatus of FIG. 6 integrated as part of a cart according to an exemplary embodiment of the present disclosure.
FIG. 14 is a perspective view of an auxiliary function control apparatus incorporated into an auxiliary system cart according to an exemplary embodiment of the present disclosure.
FIG. 15 is an enlarged view of an exemplary embodiment of auxiliary function units and user interface overlay panel of FIG. 14.
FIG. 16 is a partial back view of the cart of FIGS. 13 and 14.
FIG. 17 is a diagrammatic view of a computer-assisted surgical system that includes an auxiliary function control apparatus in accordance with an exemplary embodiment of the present disclosure.
FIG. 18 is a schematic plan view of a user interface overlay panel in accordance with an exemplary embodiment of the present disclosure.

### DETAILED DESCRIPTION

The present disclosure contemplates various auxiliary function control apparatuses that seek to optimize the arrangement and use of multiple auxiliary function units used during surgery.

As used herein, the terms "surgical" or "surgery" as used to describe various procedures and instruments may refer to a variety of procedures used for manipulation of body parts (e.g., suturing, ablating, cutting grasping, fulgurating, cauterizing, stapling, etc.), but can also include imaging, sensing, diagnostic, therapeutic, and other similar procedures. Thus, for example, an endoscopic imaging device may be considered a surgical instrument within the context of the present disclosure. The term medical device includes both surgical instruments, as well as other devices, such as irrigation, insufflation, and evacuation devices that are used in surgical procedures.

The present disclosure contemplates various embodiments of auxiliary function control apparatuses that include an arrangement of two or more auxiliary function units that are each configured to be operably connected to a corresponding one or more medical devices to provide auxiliary function control of such medical devices during a surgical procedure. Various auxiliary function units are used in surgical procedures to provide control over such medical devices other than motion control over a medical device, including, for example, an end effector of a surgical instrument. Examples of auxiliary function units used to operably connect to and control auxiliary functions of a medical device, include, but are not limited to insufflation and evacuation units, electro-surgical energy generation units (sometimes referred to as ESUs), endoscopic imaging units, irrigation units, ultrasound units, and laser or other light generation units. These auxiliary function units typically include a user interface control portion that is used to provide input settings to the unit (e.g., function on/off, function power level, function type, function timing, etc.) and one or more connector interfaces that are used to couple the unit to one or more corresponding medical devices. A single auxiliary function unit may supply one or more than one auxiliary function. For example, a single ESU may provide monopolar energy to one or two monopolar instruments and to one or two bipolar instruments, or a single insufflation/suction unit may provide both insufflation and gas evacuation via separate gas lines. If a single auxiliary function unit supplies and controls more than one auxiliary function, each individual auxiliary function unit typically has a separate user interface control portions on the auxiliary function unit to adjust the control settings of the auxiliary function unit.

In accordance with various exemplary embodiments, auxiliary function control apparatuses include two or more auxiliary function units and a user interface overlay panel that overlays all or a portion of each of the two or more auxiliary function units. The user interface overlay panel has a user interface control portion operably coupled to and overlaying at least a portion of each of the auxiliary function units. In various embodiments, the user interface control portion is part of a single, monolithic overlay panel. In other embodiments, the user interface control portion is made of two or more component panels positioned flush and abutting one another to provide to a user a façade of a continuous user interface control portion associated with the two or more auxiliary function units. Thus, in various embodiments, an overlay panel can be a single monolithic structure or may have multiple portions abutted together in a flush manner to form a continuous outer façade for a user.

The user interface control portion of a user interface overlay panel in accordance with the present disclosure includes two or more discrete user interface control areas, each of which corresponds to and provides control settings for one of the auxiliary function units associated with the user interface control portion of the overlay panel. Similarly, each of the two or more discrete user interface control areas can be divided into two or more discrete user interface control subareas that each corresponds to an individual function of an associated auxiliary function unit. If an auxiliary function unit provides a single function, then only a single user interface control area may be needed to control the function. And if an auxiliary function unit provides more than one auxiliary function, then more than one user interface control subareas may be used to control each auxiliary function. Optionally, however, control of a single auxiliary function unit's two or more functions may be done within a single user interface control area.

A first discrete user interface control area of the user interface control portion of a user interface overlay panel transmission lines is aligned (e.g., horizontally, vertically) with a first connector or group of connectors of a first auxiliary function unit over at least a portion of which the user interface overlay panel is disposed. The first discrete user interface control area is used to control the settings, and thus auxiliary function output, at the first connector or group of connectors to thereby control the auxiliary function of a medical device coupled to the first connector (or multiple medical devices coupled to a first group of connectors). Likewise, a second, third, etc. discrete user interface control area of the user interface portion of the overlay panel is/are aligned with connectors on respective second, third, etc. auxiliary function units over which at least a portion of which the overlay panel is disposed. Similarly, if any of the two or more auxiliary function units provides more than one function from additional connectors, then corresponding user interface control subareas can generally align with the additional connector(s) and be used to provide settings adjustment to control the outputs to medical devices coupled to the respective additional connector(s).

Aside from providing control settings, any of the user interface control areas or subareas can provide settings information to a user (e.g., function power level, function on/off, function type, function timing, etc.) and/or may provide feedback to a user on a status of a medical device connected to a respective connector that is controlled by the user interface control area or subarea (e.g., medical device type, status, number of uses, etc.).

The user interface control portion of the user interface overlay panel has various input mechanisms a user can interact with to provide input to alter control settings of the associated auxiliary function units. While such input mechanisms can be mechanical components (e.g., knobs, buttons, dials, etc.), in various exemplary embodiments the user interface control portion comprises a graphical user interface display utilizing touchscreen technology. The user interface overlay panel may be configured as a touchscreen display with corresponding touchscreen technology disposed as part of or coupled to the overlay panel. It is further contemplated that a user interface control portion can have a combination of mechanical input mechanisms and a graphical user interface, either for providing feedback to a user and/or including, for example, providing a touchscreen graphical user interface input mechanism.

The user interface overlay panel, including the user interface control portion, and the corresponding individual auxiliary function units, can be arranged so as to facilitate the overall use of the various auxiliary function units and to achieve an integrated apparatus that provides a simplified user operation and experience. In this manner, the disclosed auxiliary function control apparatuses provide an integrated hub that optimizes the control settings and connector layout of the individual auxiliary function units used during a surgical procedure. The disclosed auxiliary function control apparatuses also provide an integrated user interface for altering control settings and receiving feedback and other information pertaining to the individual auxiliary function units and the respective medical devices to which they are connected.

In disclosed aspects, two or more auxiliary function units are arranged with reference to the user interface control portion of a user interface overlay panel such that transmission lines (e.g., electrical transmission lines and wires, fluid lines, data lines, etc.) coupled to the connector interfaces on the auxiliary function units do not interfere with user interface control areas on the panel. In some embodiments, one or more of the connector interfaces are positioned around an edge of the user interface overlay panel, such as along a side, bottom, and/or top edge of the panel. According to the invention, the user interface overlay panel extends over portions of the two or more auxiliary function units that include the connector interfaces. One or more ports in the panel are aligned with the one or more connectors to allow one or more connector interfaces on the lines of medical devices to be coupled to the connector interfaces through the ports in the overlay panel.

In disclosed aspects, an auxiliary function control apparatus according to the present invention is integrated into a portable apparatus that facilitates transport of the apparatus, for example, within an operating room or into and out of an operating room. In this way, the task of moving multiple separate auxiliary function units and/or stocking rooms with multiple sets of auxiliary function units can be eliminated or minimized. The auxiliary function control apparatus is integrated as part of a single movable cart to provide portability. To hold the multiple auxiliary function units in a desired arrangement, e.g., a stacked arrangement, with respect to the user interface control portion of the overlay panel, shelves may be used and may be accessible from a side of the cart that does not interfere with the user interface control portion.

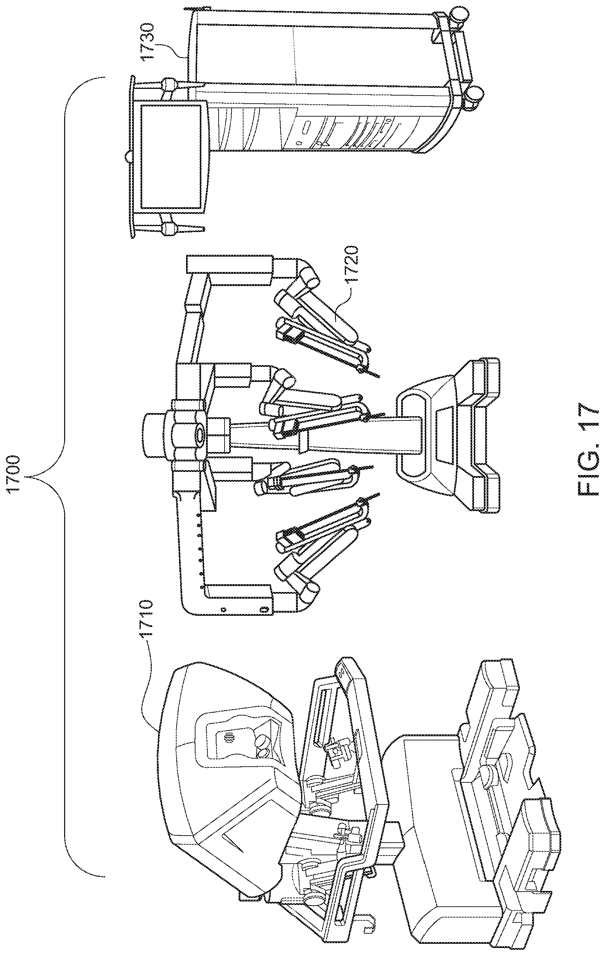

In this manner, exemplary embodiments discussed herein can be used in support of a surgical procedure, for example, to organize and control the various auxiliary function units associated with the surgical procedure. Exemplary embodiments described herein may be used, for example, with computer-assisted surgical systems (sometimes referred to as robotic surgical systems) such as, but not limited to, the da Vinci Xi^{®}, da Vinci X^{®}, and da Vinci SP^{®} Surgical Systems commercialized by Intuitive Surgical, Inc. Reference is made to FIG. 17 diagrammatically illustrating the main system components of one exemplary embodiment of a computer-assisted surgical system 1700 including a system user control unit 1710, which may be a surgeon console as shown in FIG. 17 but may have other forms as well. The system 1700 may further include a manipulating system 1720 and an auxiliary system 1730. The present disclosure describes various embodiments of auxiliary function control apparatuses that can be used as the auxiliary system 1730 shown in FIG. 17. The specifics of the system components 1710 and 1720 can vary and are illustrated as one embodiment of a surgical system with which the auxiliary function control apparatuses disclosed herein may be used.

However, use with such systems or with computer-assisted surgical systems is not limiting of the scope of the disclosure, and the auxiliary function control apparatuses disclosed herein can be used in conjunction with manual surgical procedures and systems as well. To this end, disclosed embodiments of auxiliary function control apparatuses may facilitate the initial, manual preparation for surgery (e.g., first entry via obturator, initial endoscopic examination) that may occur before the transitioning to the use of a computer-assisted surgical procedure.

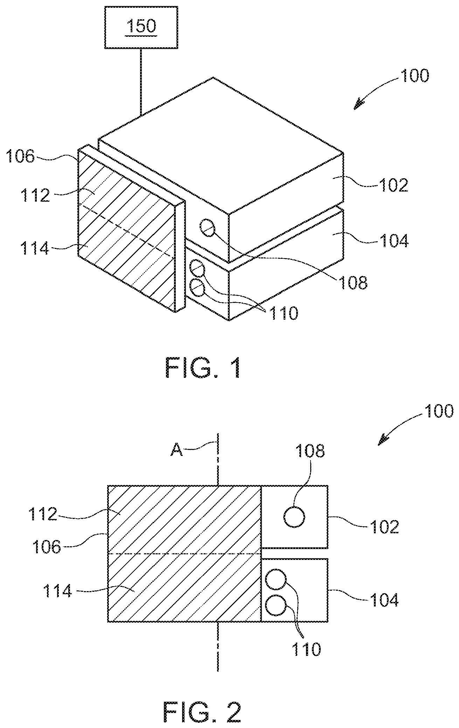

Referring now to FIGS. 1 and 2, a side perspective and front plan view of an exemplary embodiment of an auxiliary function control apparatus 100 is shown. As illustrated in FIGS. 1 and 2, the auxiliary function control apparatus 100 includes a first auxiliary function unit 102, a second auxiliary function unit 104, and a user interface overlay panel 106 that overlays a portion of both the first and second auxiliary function units 102 and 104. The first and second auxiliary function units 102 and 104 are each configured to connect to and control auxiliary functions of medical devices during a surgical procedure, as has been explained above. A first auxiliary function performed by one or more medical devices can be controlled by the first auxiliary function unit 102, and a second auxiliary function performed by one or more medical devices can be controlled by the second auxiliary function unit 104. In accordance with various exemplary embodiments, as will be described further below, the first and second auxiliary functions differ from each other, and may include, but are not limited to, electrical energy delivery, fluid delivery, fluid suction, optical imaging and/or laser or other light generation. In this regard, the first auxiliary function unit 102 includes one or more first connector interfaces 108 (one connector interface 108 being shown in the exemplary embodiment of FIGS. 1 and 2) configured to be operably connected to one or more medical devices via mating engagement with one or more first transmission lines (not shown). The second auxiliary function unit 104 includes one or more second connector interfaces 110 (two connector interfaces 110 being shown in the exemplary embodiment of FIGS. 1 and 2) configured to be operably connected to one or more medical devices via mating engagement with one or more second transmission lines (not shown). Those having ordinary skill in the art would appreciate that each of the first and second auxiliary function units can have any number of connector interfaces, and the number may depend on the auxiliary function and instruments to be controlled by the auxiliary function unit.

The auxiliary function control apparatus 100 also includes a computer controller 150 that is operably coupled to the first auxiliary function unit 102, the second auxiliary function unit 104, and to the user interface overlay panel 106 to control the user interface portion of the panel 106, which will be described in more detail below. The computer controller 150 can, for example, store software used to operate the first and second auxiliary function units 102, 104 and the panel 106, including for example, graphics and/or touch screen technology of the panel 106, as well as coordinating input settings adjustments at the panel 106 to control of the output of the connector interface of the auxiliary function units 102, 104. Software can include computer programs, firmware, or some other form of machine-readable instructions, including an operating system, utilities, drivers, network interfaces, applications, and the like. The controller 150 can further include one or more computer processing elements such as a microprocessor or other circuitry to retrieve and execute software from the computer controller 150. The computer controller 150 can also comprise other components such as one or more power management unit, one or more control interface units, one or more data storage device, etc.

The user interface overlay panel 106 comprises a user interface control portion on at least a portion of the front face thereof. The user interface control portion is arranged to serve as a user interface to allow user input and feedback to provide settings controls and information for both the first and second auxiliary function units 102, 104. In this way a simplified look and feel is provided to a user as compared to providing separate auxiliary function units each with its own separate user interface control portion. In accordance with exemplary embodiments, the user interface control portion of the overlay panel 106 is divided into a first user interface control area 112 and a second user interface control 114 that are discrete from each other in terms of the control and information settings displayed. In this way, the first auxiliary function unit 102 is controlled in response to input at the first user interface control area 112 and the second auxiliary function unit 104 is controlled in response to input at the second user interface control area 114. As illustrated in FIG. 1, the first user interface control area 112 is arranged to align with the first connector interface 108 and the second user interface control area 114 is arranged to align with the second connecter interfaces 110. As discussed above, the first and second user interface control areas may be provided as graphical user interface displays that are touchscreen enabled.

In accordance with various exemplary embodiments, as shown in FIG. 2, the first and second auxiliary function units 102 and 104 are disposed in a stacked arrangement along an axis A (*e.g.*, vertical stack in the orientation of FIG. 2) of the apparatus 100 and the user interface overlay panel 106 is positioned relative to the stacked auxiliary function units 102 and 104 such that the first user interface control area 112 overlays at least a portion of the first auxiliary function unit 102, and the second user interface control area 114 overlays at least a portion of the second auxiliary function unit 104. As illustrated in FIGS. 1 and 2, the first user interface control area 112 overlays a portion of the first auxiliary function unit 102 that is directly adjacent to and generally aligned with the first connector interface 108. The second user interface control portion 114 overlays a portion of the second auxiliary function unit 104 that is directly adjacent to and aligned with the second connector interfaces 108, 110. In this manner, the connector interfaces 108 and 110 are left exposed (*i.e.*, the connector interfaces 108 and 110 are not covered by the overlay panel 106) and provide for easy connection to instruments through transmission lines, as described above.

Various embodiments of the present disclosure further contemplate arranging auxiliary function units, and their respective connector interfaces, and the user interface overlay panel such that transmission lines providing mating connection between the connector interfaces and connected medical devices do not obstruct viewing of the user interface control portion. As illustrated in FIGS. 1 and 2, for example, the first and second auxiliary function units 102 and 104 are disposed in the stacked arrangement such that the connector interfaces 108 and 110 are positioned such that, when mated with the respective transmission lines, the transmission lines do not overlay or obscure the front face, viewable portion of the overlay panel 106 that includes the user interface control portion, but rather can be connected to the connector interfaces 106, 108, 110 and routed in a direction away from the front face and user interface control portion and to the connected medical devices.

Those of ordinary skill in the art will understand, however, that the auxiliary function control apparatus 100 illustrated in FIGS. 1 and 2 and described above is exemplary only and that auxiliary function control apparatuses in accordance with the present disclosure may have various types, configurations, and/or arrangements of auxiliary function units, with various types, numbers and/or configurations of auxiliary function connector interfaces, without departing from the scope of the present disclosure and claims. In this regard, in accordance with various disclosed embodiments selection of the relative stacked arrangement of the auxiliary function units may be altered depending on the arrangement of the connector interfaces and with consideration of how the connector interfaces will be connected through transmission lines to various medical devices used in the surgical field.

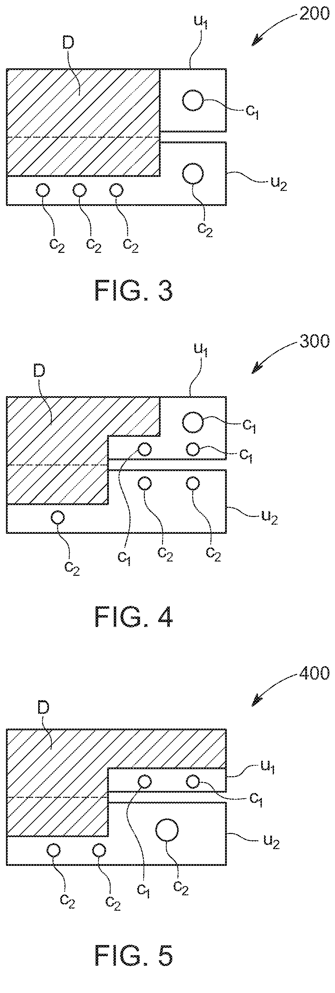

Accordingly, based on a given arrangement of auxiliary function units and connector interfaces, auxiliary function control apparatuses in accordance with the present disclosure may also have various types, shapes and/or sizes of user interface overlay panels with user interface control portions. FIGS 1-10 illustrate various exemplary auxiliary function control apparatuses 100, 200, 300, 400, 500, 600, 700, 800, 900, 1000 having differing numbers and arrangements of auxiliary function units with differing numbers and arrangements of connector interfaces and how the user interface control portion of a user interface overlay panel can be positioned to meet the design criteria discussed above. In various embodiments, for example, the user interface overlay panel and/or its user interface control portion has a generally rectangular shape (see, e.g., FIGS. 1-3, 6, 7, and 9-12), whereas in various additional embodiments the panel and/or user interface control portion of the panel may have a different polygonal shape (see, e.g., FIGS. 4, 5, and 8). Those having ordinary skill in the art would appreciate that rectangular or other polygonal shapes can include rounded corners. For ease of disclosure, in the schematic representations of the embodiments of FIGS. 3-5 and 8-10, the auxiliary function units are labeled Uₙ, the connector interfaces are labeled Cₙ, and the user interface overlay panels having user interface control portions in the form of graphical user interface displays are labeled D, where n is a numerical identifier intended to distinguish between the auxiliary function units and their respective connectors. Based on the disclosure of the present application, those of ordinary skill in the art will understand how to select various arrangements and configurations of the overlay panel having the graphical user interface control portion, with respect to the stacked auxiliary function units, for a given application.

Those of ordinary skill in the art will further understand that auxiliary function control apparatuses in accordance with the present disclosure may include various numbers of auxiliary function units for controlling various types of auxiliary functions that are utilized during a surgical procedure, and which are stacked in various arrangements. For example, an auxiliary function control apparatus that combines control of imaging functionality in combination with two other differing types of auxiliary functions, such as, for example, suction/irrigation functionality and electrosurgical energy functionality, may be useful in various surgical applications. In such an application, at least three differing types of auxiliary function units may be used, *i.e*., an imaging function unit, an electrosurgical energy function unit, a suction/irrigation function unit. Further, in some embodiments, it may be desirable to provide two imaging function units in combination with an electrosurgical energy function unit and a suction/irrigation function unit. Those having ordinary skill in the art would appreciate that the numbers and combinations above are non-limiting.

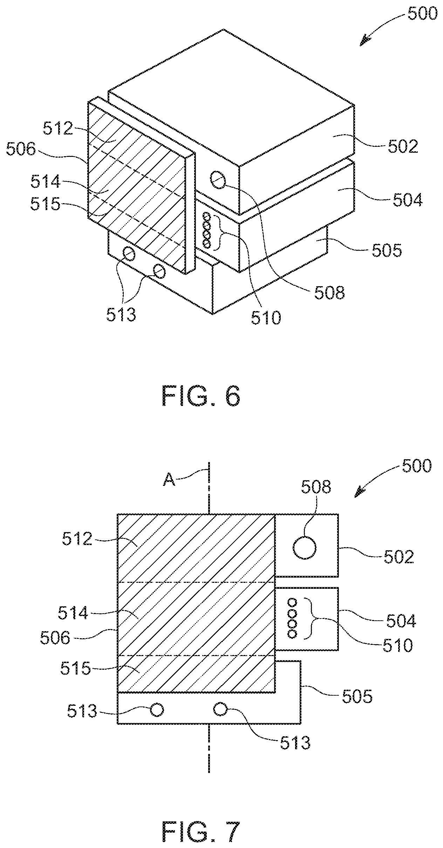

Thus, as illustrated in the embodiments of FIGS. 6-12, auxiliary function control apparatuses having three types of auxiliary function units in a stacked arrangement are shown. Referring to the embodiment of FIGS. 6 and 7, an auxiliary function control apparatus 500 includes a first auxiliary function unit 502, a second auxiliary function unit 504, a third auxiliary function unit 505. A user interface overlay panel 506 having a user interface control portion is operably connected to each of the first, second, and third auxiliary function units 502, 504, and 505.

As discussed above with reference to the embodiment of FIGs. 1 and 2, a different auxiliary function is controlled by each of the auxiliary function units 502, 504, and 505, such that a first auxiliary function is controlled by the first auxiliary function unit 502, a second auxiliary function is controlled by the second auxiliary function unit 504, and a third auxiliary function is controlled by the third auxiliary function unit 505. Thus, similarly, the first auxiliary function unit 502 includes one or more first connector interfaces 508 (one connector interface 508 being shown in the exemplary embodiment of FIGS. 6 and 7) configured to be operably connected to one or more medical devices via mating engagement with one or more transmission lines (not shown). The second auxiliary function unit 504 includes one or more second connector interfaces 510 (four connector interfaces 510 being shown in the exemplary embodiment of FIGS. 6 and 7) configured to be operably connected to one or more medical devices via mating engagement with one or more second transmission lines (not shown). And, the third auxiliary function unit 505 includes one or more third connector interfaces 513 (two connector interfaces 513 being shown in the exemplary embodiment of FIGS. 6 and 7), which are configured to be operably connected to one or more medical devices via mating engagement with one or more third transmission lines (not shown).

Similar to the embodiment of FIGS. 1 and 2, the user interface overlay panel 506 is arranged to overlay at least a portion of each of the auxiliary function units 502, 504, and 505. The user interface control portion of the overlay panel 506 is divided into a first user interface control area 512, a second user interface control area 514, and a third user interface control area 515, wherein each of the first, second, and third user interface control areas is arranged to align with a respective one of the first, second, and third auxiliary function connector interfaces 508, 510, and 513. The first auxiliary function unit 502 is controlled in response to input at the first user interface control area 512, the second auxiliary function unit 504 is controlled in response to input at the second user interface control area 514, and the third auxiliary function unit 505 is controlled in response to input at the third user interface control area 515. Although not shown, those having ordinary skill in the art would appreciate based on the present disclosure that any of the user interface control areas 512, 514, 515 can have further control subareas to control the output of different connector interfaces of the corresponding auxiliary function unit. For example, different user interface subareas of user interface control area 514 may provide control settings and information for the different connector interfaces or sets of connector interfaces 510, and likewise with user interface control area 515 and the connector interfaces 513.

As shown in FIG. 7, the first, second, and third auxiliary function units 502, 504, and 505 are disposed in a stacked arrangement along an axis A of the apparatus 500 (*i.e.,* the vertical direction in the orientation of the figures). The user interface overlay panel 506 is positioned relative to the stacked auxiliary function units 502, 504, and 505 such that each of the first, second, and third user interface control areas 512, 514, and 515 overlays at least a portion of the respective first, second, and third auxiliary function units 502, 504, and 505.

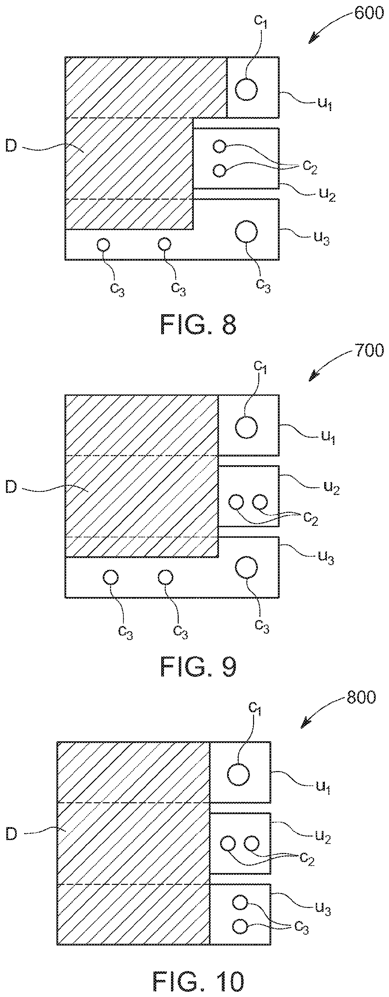

As above, those of ordinary skill in the art would understand that the auxiliary function control apparatus 500 illustrated in FIGS. 6 and 7 and described above is exemplary only and that auxiliary function control apparatuses in accordance with the present disclosure may have various configurations, as illustrated, for example, by exemplary auxiliary function control apparatuses 600, 700, and 800 of FIGS. 8-10 (containing reference labeling similar to that described in connection with the embodiments of FIGS. 3-5) without departing from the scope of the present disclosure and claims.

Although auxiliary function control apparatuses of various exemplary embodiments shown in the figures have two or three auxiliary function units, those having ordinary skill in the art would understand that those numbers are non-limiting and any number of auxiliary function units may be used with a continued graphical user interface display arranged in accordance with the principles disclosed herein. For example, those of ordinary skill in the art would appreciate that any of the auxiliary function units arranged in a stack could be provided as two or more auxiliary control units of the same type in a layer of the stack, and the arrangement of the user interface control areas would still generally be aligned to provide control of the multiple auxiliary function units of the same type in a layer of the stack.

Moreover, those of ordinary skill in the art would appreciate that the axis A could extend perpendicularly to the orientation shown in the figures such that the arrangement of the stacked auxiliary function units and relative orientation of the continuous graphical user interface display is rotated 90 degrees either clockwise or counterclockwise, in which case the stacked arrangement of auxiliary function units would be horizontally stacked in the views of the figures. Likewise, the orientations of the auxiliary function control apparatuses may be rotated 180 degrees from the orientations shown in the figures. If such arrangements are implemented, placement of the auxiliary function control apparatus may be selected so as to ensure that transmission lines providing connection between the connector interfaces of the auxiliary function units and medical devices to be used in the surgical procedure do not obstruct the view of the display; in other words, such transmission lines should extend from the connector interfaces without draping over user interface control areas.

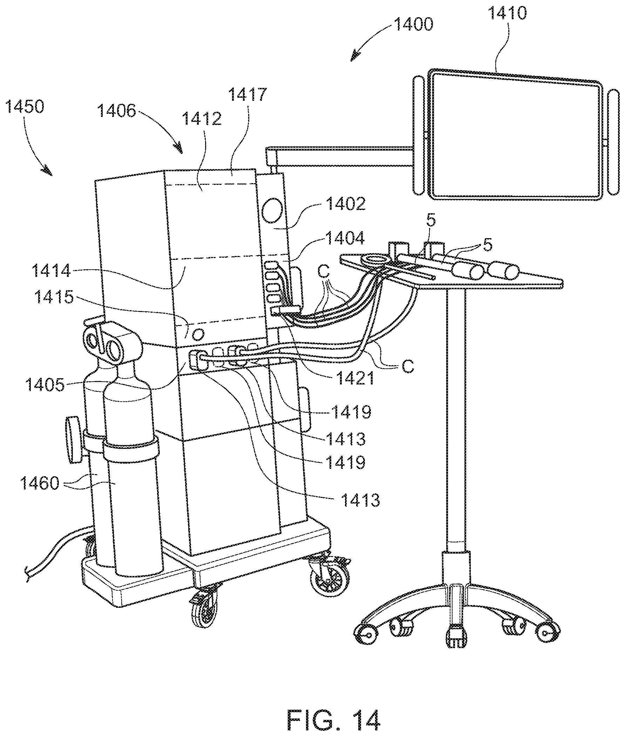

In the embodiments of FIGS 1-10, the connector interfaces of each of the apparatuses 100, 200, 300, 400, 500, 600, and 800 are not covered by the user interface overlay panel, but are left exposed for connection to respective transmission lines so as to operably connect to medical devices (reference is made to FIG. 14 showing the connection of connector interfaces 1410, 1413 of the auxiliary function units 1404 and 1405 to various surgical instruments S through transmission lines C). According to the present invention the auxiliary function control apparatuses include a user interface overlay panel that extends beyond the user interface control portion and overlays the connector interfaces of the auxiliary function units. Such a user interface overlay panel that overlays the connector interfaces of the auxiliary function units includes connector port openings that can receive connectors on transmission lines to ultimately place the transmission lines in operable connection with the connector interfaces on the auxiliary function units so as to operably connect the auxiliary function units with medical devices via the transmission lines.

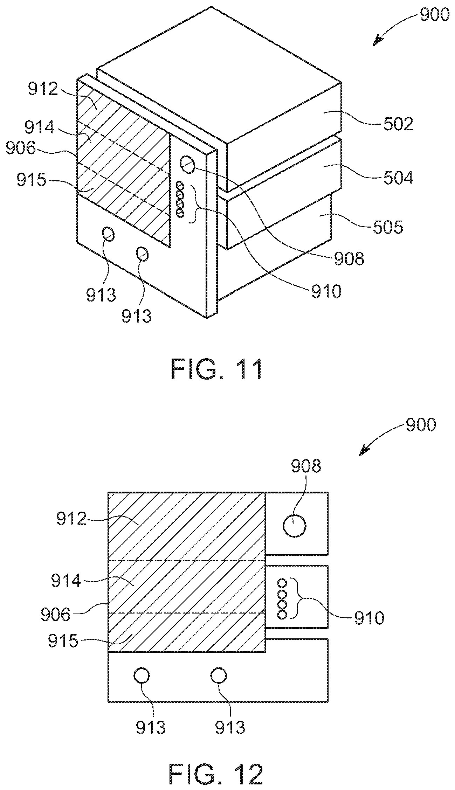

With reference to an embodiment of the present invention of FIGS. 11 and 12, schematically depicting a side perspective view and a front plan view, an auxiliary function control apparatus 900 includes the stacked arrangement of the first, second, and third auxiliary function units 502, 504, and 505 of FIGS. 6 and 7 and a user interface overlay panel 906 that is operably coupled to each of the auxiliary function units 502, 504, and 505. As illustrated in FIGS. 11 and 12, the user interface overlay panel 906 extends beyond the user interface control portion comprising the various user interface control areas 912, 914, 915 so as to overlay the connector interfaces 508, 510, and 513 (not visible in FIGS. 11 and 12) of the auxiliary function units 502, 504, and 505. The user interface overlay panel 906 is also configured to receive the connectors of transmission lines to enable the transmission lines, and thus medical devices to which the transmission lines are connected, in mating engagement with the connector interfaces 508, 510, and 513 of the auxiliary function units 502, 504, 505.

The user interface overlay panel 906 includes one or more first port openings 908 (one first port opening 908 being shown in the embodiment of FIGS. 11 and 12 to correspond with the one first auxiliary function connector interface 508 of the auxiliary function unit 502) overlying the one or more first auxiliary function connector interfaces 508; one or more second port openings 910 (four second port openings 910 being shown in the embodiment of FIGS. 11 and 12 to correspond with the four second auxiliary function connector interfaces 510 of the auxiliary function unit 504) overlying the one or more second auxiliary function connector interfaces 510; and one or more third port openings 913 (two third port openings 913 being shown in the embodiment of FIGS. 11 and 12 to correspond with the two third auxiliary function connector interfaces 513 of the auxiliary function unit 505) overlying the one or more third auxiliary function connector interfaces 513. The first, second, and third port openings 908, 910, and 913 are configured to receive the connectors of transmission lines and place those connectors into mating engagement with the respective first, second, and third auxiliary connector interfaces 508, 510, and 513.

The port openings 908, 910, 913 are arranged on the user interface overlay panel 906 to be directly aligned with the corresponding auxiliary function unit connector interfaces 508, 510, 513. Like the connector interfaces, the port openings may include various types, numbers and/or configurations of openings without departing from the scope of the present disclosure and claims.

In addition, as will be described further below, in various embodiments it is contemplated that the user interface overlay panel that covers the portions of the auxiliary function units that include the connector interfaces can also include user interface control areas and/or subareas in addition to those provided on the portion of the user interface overlay panel that is disposed adjacent to the connector interfaces.

As has been described above, the various user interface control portions of the overlay panel in any of the embodiments described can be graphical user interface displays that include touchscreen technology.

A user interface overlay panel in accordance with the present disclosure, may include various features and indicators (*e.g*., visual indicators) to assist users with using the auxiliary function units and in making a proper connection between transmission lines and the connector interfaces of the auxiliary function units. In various embodiments, the port openings are configured to light up when a proper connection is made to a medical device to be controlled by the auxiliary function unit, for example by way of a transmission line providing electrical, data, and/or other flux transmission connection between the two. In an embodiment, each of the auxiliary function units can have a distinct color code assigned to the unit, and the port openings associated with each auxiliary function unit may be configured to light up, via, for example, an associated light ring with the assigned color to provide an immediate indication of connection status. In another embodiment, the color of the light, regardless of the auxiliary function unit type, may indicate either a proper connection or an improper or null connection state (*e.g.,* green for proper connection state and red for improper or null connection state). In various additional embodiments, as instruments are connected to the respective auxiliary function units of the auxiliary function control apparatus, a confirmation light (*e.g*., an LED) may illuminate along with a responsive user interface control area/subarea (*e.g.,* displayed on a corresponding graphical user interface portion of the user interface overlay panel (see FIG. 14)) that conveys information to instruct users through setup and/or other important information related to the auxiliary function unit and/or medical devices.

User interface overlay panels also can include various others user interface features, such as, for example, power switches, speakers, microphones, keypads, etc. to allow a user to provide input and receive feedback related to control of the overall auxiliary function control apparatus and auxiliary function units.

As discussed above, in accordance with various exemplary embodiments, auxiliary function control apparatuses according to various embodiments of the present disclosure can, for example, organize multiple auxiliary function units in a stacked arrangement within a cart to provide ease of transport within, and into and out of, an operating room. In an exemplary embodiment, the auxiliary function control apparatus can be the auxiliary system of a computer-assisted surgical system that employs robotic technology.

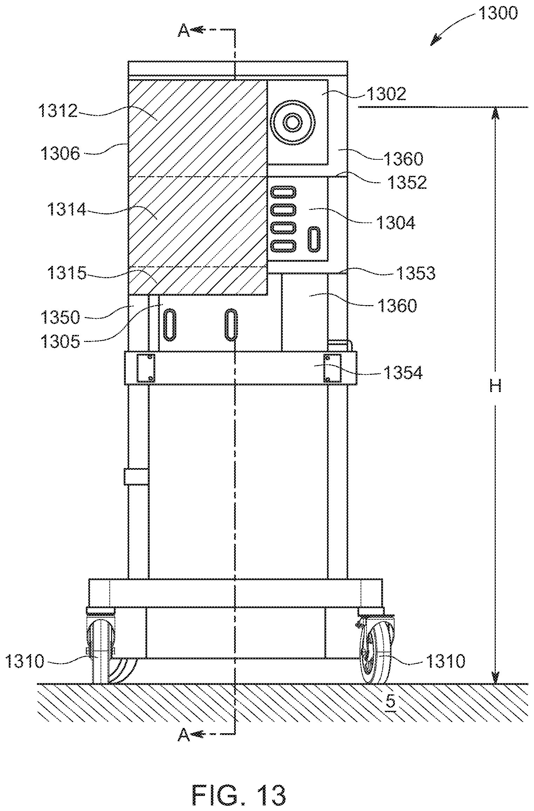

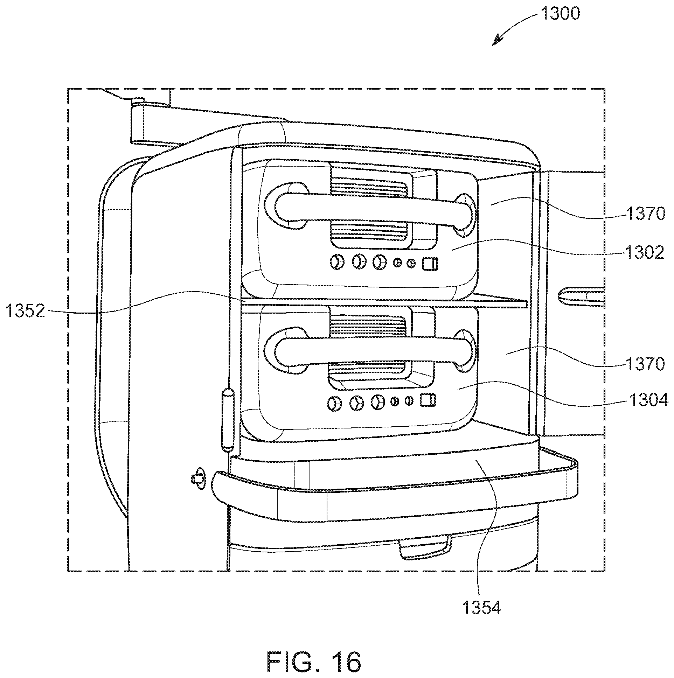

With reference now to FIG. 13, according to the present invention, the auxiliary function control apparatus 1300 includes a cart 1350 preferably having wheels 1340 and shelves 1352, 1353, and 1354 respectively holding three auxiliary function units 1302, 1304, and 1305 in a stacked arrangement. As shown in FIG. 13, in such an arrangement, the user interface overlay panel 1306 overlies openings 1360 on one side of the shelves 1352, 1353, and 1354. The auxiliary function units 1302, 1304, and 1305 are removable through openings 1370 on another side, such as, for example, an opposite side of the openings 1360, as shown in FIG. 16 (which only depicts two shelves 1352, 1354 and two auxiliary function units). Although not shown, the cart 1350 may include a door or other cover that can close over the openings 1370 through which the auxiliary function units 1302, 1304, 1305 can be removed to provide protection and secure closure of the auxiliary function units when the auxiliary function control apparatus is in use or is being moved. Thus, the auxiliary function units can be field replaceable for maintenance, replacement, and service. Although not shown in FIG. 13, the cart 1350 can further include components of conventional auxiliary systems of a teleoperated surgical system, such as, for example, a display that presents a view of the surgical field obtained from an imaging device telestration, as well as other controllers and/or processors that provide control of an overall surgical system. Those having ordinary skill in the art would be familiar with other components that may be provided on such a cart.

As discussed above, various exemplary embodiments of an auxiliary function control apparatus may integrate as the auxiliary function units at least an insufflation and evacuation function unit and an imaging function unit (e.g., configured to provide illumination and endoscopic imaging), which are generally used in a variety of surgical procedures. Providing an electrosurgical energy control unit (ESU) can also be useful as a third auxiliary function unit. Such a combination of auxiliary functions may have application in a variety of surgical procedures, such as for example, in a variety of surgical procedures that are performed using computer-assisted surgical systems employing robotic technology.

When an insufflation and evacuation function unit is one of the auxiliary function units of an auxiliary function control apparatus according to various embodiments of the present disclosure, the overall arrangement of the auxiliary function units may take into account the height at which the hose connector interface of the insufflation and evacuation function unit is disposed. This factor may be taken into consideration along with the criteria regarding providing and aligning a user interface control portion comprising user interface control areas/subareas and arranging the connector interfaces of the auxiliary function units to avoid obstruction of the user interface control portion when connecting transmission lines thereto described above for various embodiments. More specifically, a height of the hose connector interface may be such that potential backflow of body fluids (e.g., from a patient) into the insufflator may be minimized. Body fluids, for example, can clog the insufflator tubeset filter (causing procedure delay while a new tubeset is installed) or even damage the insufflator if any fluid gets past the tubeset filter. Thus, in accordance with various embodiments, to minimize the potential for the backflow of body fluids, the insuflattor is kept high relative to the patient. In various embodiments, for example, the hose connector interface is at a height H of about 48-55 inches (122-140 cm), for example about 53 inches (135 cm), above a ground surface.

To account for this desired height of the insufflation hose connector interface, various embodiments of an auxiliary function control apparatus include a stacked arrangement of the auxiliary function units such that the insufflation and evacuation function unit is arranged above other auxiliary function units, for example in a vertically stacked arrangement of the auxiliary function units as described with reference to various embodiments of the figures herein for example. In this way, the heights of the various user interface control areas and associated auxiliary connector interfaces with which a user interacts can be within an ergonomic height range for a wide range of individuals. In a non-limiting exemplary embodiment, auxiliary connector interfaces can be positioned at a height of greater than about 30 inches (76 cm), the user interface control portion can be positioned at a height of greater than about 41 inches (104 cm), and the overall height of an auxiliary function control apparatus, including one that has a cart, may be less than about 60 inches (152 cm).

FIG. 14 shows one exemplary embodiment of an auxiliary function control apparatus 1400 that integrates an insufflation and evacuation function unit, one or more endoscopic imaging function units 1405, and an electrosurgical energy function unit (ESU) with a continuous graphical user interface display as part of a transportable cart. As illustrated in the embodiment of FIG. 14, the auxiliary function control apparatus 1400 includes a surgical insufflation and evacuation function unit 1402 having an auxiliary function connector interface 1408 configured to receive an insufflator hose, an ESU 1404 having four energy connector interfaces 1410, and an endoscopic imaging function unit 1405 having two endoscope connector interfaces 1413. Alternatively, the endoscopic imaging function unit 1405 may be provided as two different units each with one endoscope connector interface 1413, as reflected by the dashed line in FIG. 14 providing a vertical separation indicating two different units (one on the left of the vertical dashed line and one on the right). Further, the one or more endoscopic imaging function units 1405, may optionally include one or more "dummy" receptacles 1419 that are sized and shaped similar to the outer perimeter of the connector interfaces 1413. Such receptacles, which are not connector interfaces and thus are not electrically "active," can provide a holster to receive a transmission line of an endoscope that is not in use, for example, if an endoscope is being switched with another. Such a receptacle can thus facilitate one-handed switching out of one endoscope for another.

The auxiliary function units are arranged in a vertical stack (in the orientation of FIG. 14) such that the insufflation and evacuation function unit 1402 is positioned over the electrosurgical energy function unit 1404, and the electrosurgical energy function unit 1404 is positioned over the endoscopic imaging function unit 1405. In an exemplary embodiment, the height of the connector interface 1408 of the insufflation and evacuation unit can range from 122 cm - 140 cm (48 in. - 55 in.), for example about 134 cm (53in.); the height of connector interfaces 1410 of the electrosurgical energy unit 1404 can range from about 104 cm - 124 cm (41 in. - 49 in.), for example about 107 cm - 119 cm (42 in. - 47 in.); - 47 in.; and the height of connector interfaces 1413 for the endoscopic imaging unit 1405 can range from about 86 cm - 94 cm (34 in. - 37 in.) for example about 89 cm (35 in.).

The auxiliary function units 1402, 1404, and 1405 can be positioned in shelves (not shown) of a wheeled cart 1450, as described with reference to the embodiment of FIG. 13 for example. This arrangement allows for the height of the insufflation hose connector interface to be sufficiently above a patient to prevent or minimize the risk of backflow, as discussed above.

As can also be seen in the embodiment of FIG. 14 and arrangement of the three auxiliary function units 1402, 1404, and 1405, the connector interfaces 1408, 1410, and 1413 are located such that when transmission lines are placed in mating engagement with the connector interfaces (labeled C for convenience in FIG. 14), they can extend to the surgical instruments to which they are connected (which are ultimately located in the surgical field) without obstructing the user interface control areas 1412, 1414, and 1415 of the user interface overlay panel 1406. In addition to the arrangement of the auxiliary function units 1402, 1404, and 1405 (*i.e.*, to prevent the transmission lines C from obstructing the user interface control areas 1412, 1414, and 1415), various embodiments may also include one or more hangers 1421 (one hanger 1421 being shown in the embodiment of FIG. 14) to hold one or more transmission lines C . As illustrated in FIG. 14, for example, the hanger 1421 may be attached to the cart 1450 and a plurality of transmission lines C can be routed through the hanger 1421 before being dispersed into the sterile field.

As further illustrated in the embodiment of FIG. 14, the electrosurgical energy connector interfaces 1410 are vertically aligned underneath the hose connector interface 1408, while the endoscope connector interfaces 1413 are disposed in a position vertically aligned with the user interface overlay panel 1406. In the embodiment of FIG. 14, the user interface overlay panel 1406 has a generally rectangular shape, with the connector interfaces 1408, 1410 disposed along a first edge 1420 of the display panel 1406, and the connector interfaces 1413 being disposed along a second edge 1430 of the display panel 1406 that is perpendicular to the first edge 1420.

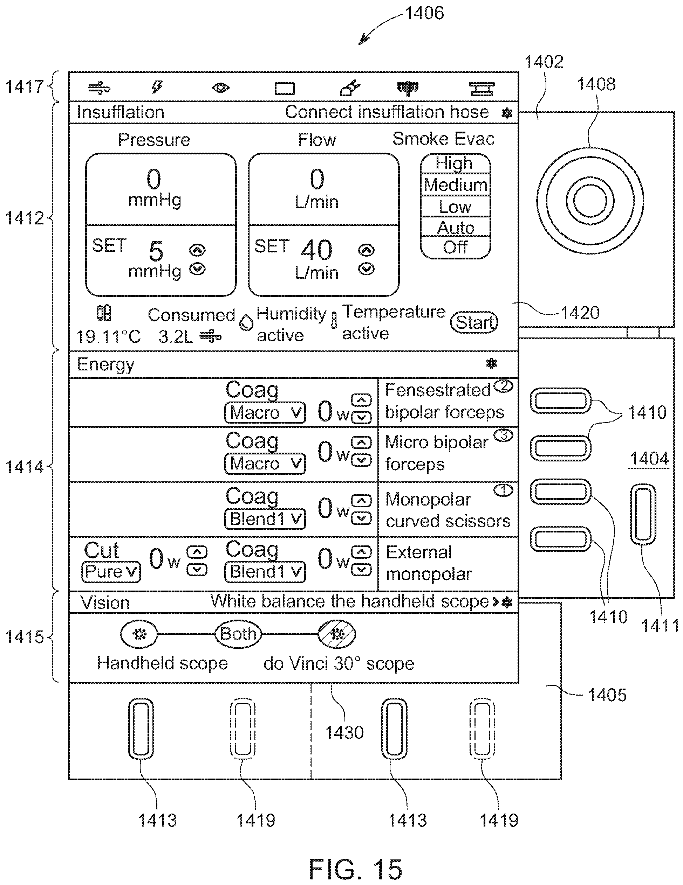

The cart 1450 of the auxiliary function control apparatus 1400 also can have a display 1410, similar to conventional auxiliary systems of other computer-assisted surgical systems. The display 1410 provides a display of the images being taken of a remote surgical site from the imaging instruments connected to the imaging function unit 1405. The cart 1450 also can include a platform or other storage area to receive tanks 1460 for supply of insufflation gas. Further, as described above with reference to FIG. 16, the auxiliary function units 1402, 1404, 1405 can be accessible from a rear of the cart 1450 (i.e., the side opposite the face shown in FIG. 14 and 15) through a door so that maintenance and/or removal and replacement of the units 1402, 1404, 1405 can readily occur.

While the user interface overlay panel 1406 shown in FIG. 14 has a configuration in which the edges 1420, 1430 terminate adjacent to the auxiliary connector interfaces 1408, 1410, 1413, according to the invention, the graphical user interface display panel 1406 extends over the connector interfaces 1408, 1410, 1413, and includes respective port openings configured to receive connectors on transmission lines to place the transmission lines in mating engagement with the respective connector interfaces 1408, 1410, 1413, as described above with reference to the embodiments of FIGS. 11 and 12 for example.

With reference to FIG. 15, the user interface overlay panel 1406 of the auxiliary function control apparatus 1400 includes various features and indicators (e.g., visual indicators) to assist users with using the auxiliary function units 1402, 1404, 1405, and in making a proper connection between the transmission lines operably coupled to surgical instruments and the connector interfaces 1408, 1410, and 1413. As illustrated in the enlarged view of FIG. 15, the user interface overlay panel 1406 is designed to collate the controls of each of the insufflation and evacuation unit 1402, ESU 1404, and imaging function unit 1405 and auxiliary connector interfaces 1408, 1410, and 1413. In this manner, as described above, the overlay panel 506 overlays at least a portion of each of the auxiliary function units 1402, 1404, and 1406, and the auxiliary connector interfaces 1408, 1410, and 1413 (or the ports of the display panel corresponding to the same in an embodiment where the display panel overlays the connector interfaces) are each aligned with a respective user interface control area 1412, 1414, and 1415 of the user interface portion of the overlay panel 1406. Each user interface control portion 1412, 1414, and 1415 includes at least one of a control user interface and a feedback user interface that corresponds to an operational state of the respective auxiliary function units 1402, 1404, and 1406 that it overlays. In the embodiment of the auxiliary function control apparatus 1400, the first user interface area 1412 is a graphical user interface that is touchscreen-enabled and includes various control settings and informational graphics (e.g., in the form icons, alphanumeric characters, and other graphical features,) that provide control over and information regarding the insufflation and evacuation function unit settings. The second graphical user interface control area 1414 also is a touchscreen-enabled graphical user interface that includes similar various control settings and informational graphics but related to control over and information regarding the ESU and associated surgical instruments operably connected thereto, such as for example one or more bipolar and/or monopolar instruments connected to appropriate connector interfaces 1410. In addition to such bipolar and/or monopolar connector interfaces 1410, an electrical ground connector interface 1411 may be provided as part of the ESU 1404, as those having ordinary skill in the art would be familiar with. The third user interface control area 1415 can include various controls and informational graphics (again, e.g., in the form of icons, alphanumeric characters, and other graphical features, including those that are touchscreen enabled similar to user interface control areas 1412 and 1414) related to control over and information regarding the endoscopic imaging unit 1405 and the endoscopes or other imaging devices operably connected thereto. As further illustrated in the embodiment of FIG. 15, the user interface overlay panel 1406 may also include a fourth user interface control area 1417 that acts as an overall system dashboard to provide at-a-glance statuses and various options for troubleshooting, instructions for use and settings of the auxiliary function control apparatus, its auxiliary function units, and connected medical devices, etc.. In this manner, the user interface control areas 1412, 1414, 1415, and 1417 may together form a universal user interface that appears on the user interface control portion of the user interface overlay panel 1406, which can also be screen-mirrored to other displays located, for example, on tablets, work stations, laptops, mobile devices, etc. used during and/or after the surgical procedure.

In various further exemplary embodiments, as above, the user interface control areas 1412, 1414, and 1415 may be configured to light up or provide another indicator when a proper connection is made between an auxiliary connector interface 508, 510, or 513 and a respective transmission line connected to a respective medical device. In various exemplary embodiments, as medical devices are connected to the auxiliary function control apparatus 1400, a confirmation light (e.g., an LED) surrounding the connector interfaces 1408, 1410, 1413 (or a port of the user interface overlay panel 1406 overlaying such a connector interface) may illuminate along with a responsive user interface control area 1412, 1414, 1415 that may, for example, provide setup or use information, and/or other information related to the auxiliary function units and/or instruments. By way of further example, if a medical device type that does not match the auxiliary function unit is attempted to be connected to the connector interfaces, the connector interfaces (or corresponding ports) will not light up and/or may turn a color indicating a connection is improper or nothing is connected.

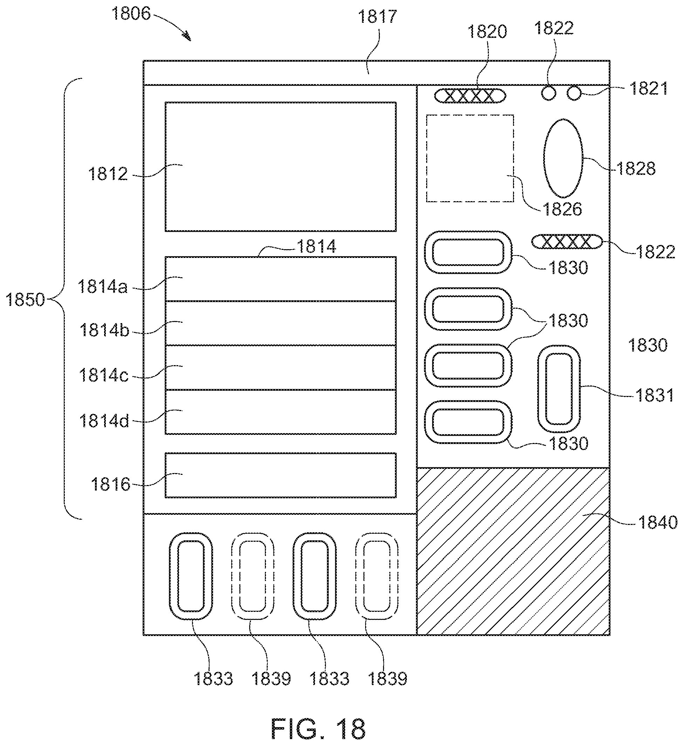

The present disclosure thus contemplates a variety of configurations and arrangements of an auxiliary function control apparatus that facilitates the overall use of the various auxiliary function units that may be used during a surgical procedure, as well as providing simplified user operation and experience of the same. One aspect of the present disclosure, as has been discussed with respect to various embodiments described herein, includes the use of a user interface overlay panel that can provide the appearance of a continuous façade that appears common to all of the auxiliary function units that are part of the auxiliary function control apparatus, and that includes an arrangement of features that enhance the user experience. One additional embodiment of such a user interface overlay panel is shown schematically and in plan view in FIG. 18. FIG. 18 illustrate an exemplary embodiment of a user interface overlay panel 1806, shown in isolation, that could be used, for example, over the arrangement of auxiliary function units depicted in FIG. 15.

The user interface overlay panel 1806 has a variety of sections and is configured to overlay the entire front areas of the auxiliary function units (not shown in FIG. 15) that are behind the overlay panel and for which the overlay panel is operably coupled to provide control. As has been described with reference to other exemplary embodiments, the overlay panel 1806 includes at least one user interface control portion 1850 that includes multiple user interface control areas 1812, 1814, 1815 corresponding to each of the auxiliary function units. More specifically, user interface control area 1812 is configured to provide control settings for and information regarding a first auxiliary function unit having a connector interface for a medical device (the connector interface not being visible and would lie behind port opening 1828). In an embodiment, the first auxiliary function unit may be an insufflation/evacuation unit as described with reference to other embodiments herein. User interface control area 1814 is configured to provide control settings for and information regarding a second auxiliary function unit having connector interfaces for medical devices (the connector interfaces not being visible and would lie behind port openings 1830, 1831). In an embodiment, the second auxiliary function unit may be an ESU as described with reference to other embodiments herein. User interface control area 1815 is configured to provide control settings for and information regarding a third auxiliary function unit having connector interfaces for medical devices (the connector interfaces not being visible and would lie behind port openings 1833, 1839). In an embodiment, the third auxiliary function unit may be an imaging, which may also include illumination functionality, as described with reference to other embodiments herein. Additionally, as described with respect to various embodiments, it may be that any of the auxiliary function units may be provided as more than one unit of the same type, for example the imaging unit may be two separate units, each with one or more connector interfaces. In such an embodiment, the user interface control areas are nevertheless arranged so as to generally be positioned to create a visual correlation to a user (e.g., by aligning with) with the respective auxiliary function unit connector interfaces that they control.

A user interface control area 1817 also may be provided that extends along all or a portion of, for example, a top edge of the user interface display panel 1806, and can provide overall system information to a user, including, for example, about various operational states of an overall surgical system that the auxiliary function control apparatus is a component.

Other sections of the user interface overlay panel 1806 can include panel portions that include the port openings to overlay the connector interfaces on the various auxiliary function units that are behind the overlay panel. Thus, the sections below and to the right of the user interface control portion 1850 as depicted in FIG. 18, have port openings 1828, 1830, 1831, 1833, 1839 that overlay so as to provide access to various connector interfaces, such as connector interfaces 1408, 1410, 1411, 1413, 1419 described with reference to the embodiment of FIG. 15. In addition to providing the port openings, those sections of the user interface overlay panel 1806 also may have additional user interface control areas, one of which is depicted as user interface control area 1826. Such areas may be useful to provide more important information or control setting capability to a user to serve as an attention-grabbing area to a user to see such pertinent setting information at a glance.

Moreover, as with other embodiments described above, any of the port openings can be surrounded with lights (e.g., LED lights) of any color to provide feedback to a user regarding connected states of the connector interfaces behind the port openings.

Additional user interface features also may be included as part of the user interface overlay panel 1806. For example, a speaker grill 1820 and microphone grill 1822 may be provided an may overlay speakers and a microphone provided as part of the auxiliary function control apparatus. Alternatively, such a speaker and microphone may be provided as parts of the overlay panel itself. In addition, an on/off button 1821 and an emergency stop button 1822 can be provided and operably connected to appropriately control power to the auxiliary function control apparatus and corresponding auxiliary function units. The layout and arrangement of these various additional features is exemplary and non-limiting, and those of ordinary skill in the art would appreciate various positions and sizes for such features can be modified and still be within the scope of the present disclosure.

Another section 1840 of the user interface overlay panel may be a customizable section that can provide a door or the like for storage in an open space behind the overlay panel 1806, or may be utilized as an additional section to provide user interface controls etc.

The various user interface control areas and subareas 1812, 1814, 1815, 1817, 1826 can be graphical user interface displays and may include touchscreen technology. As such, in at least areas behind those sections, the overlay panel may include a touchscreen interface and printed circuit board behind those sections. This would similarly apply to any of the touchscreen enabled portions of the overlay panels described with reference to other embodiments.

The user interface overlay panel 1806 can be made as a monolithic structure or may be separate panel sections that are fit together in an abutting and flush arrangement that appears as a generally continuous and common façade to a user. Such an arrangement may show slight seams where sections come together, but nevertheless would provide an integrated overall panel structure.

In various embodiments to provide additional ease of use, matching colors may be used on portions of the system. For example, the connector interfaces of each auxiliary function unit may be provided with one or more unique colors that distinguish them from the connector interfaces of other auxiliary function units of an apparatus. Those same colors may be employed for transmission lines to connect the various medical devices to the respective auxiliary function units. Moreover, in various exemplary embodiments, the colors provided on the respective user interface control areas and/or ports may match those of the corresponding connector interfaces with which they align and for which they are configured to control the settings.

Those of ordinary skill in the art will understand that the various auxiliary function control apparatuses illustrated and described with reference to the figures are nonlimiting and that other types, configurations, and/or arrangements of auxiliary function units, with various types, numbers and/or configurations of connector interfaces configured to connect to transmission lines may be envisioned without departing from the scope of the present disclosure and claims. Furthermore, based on a given surgical, therapeutic, diagnostic, etc. application, those of ordinary skill in the art will be able to determine other layouts for the auxiliary function connector interfaces and user interface overlay panel, and corresponding user interface control areas/subareas, using, for example, the following design considerations: (1) maintaining a façade that appears continuous to a user for at least the user interface control portion display panel; (2) providing observable mapping or alignment from each auxiliary function connector interface to its user interface control area/subarea of the user interface control portion of the overlay panel; (3) providing an ergonomic desired height for each of the auxiliary function connector interfaces and the user interface overlay panel; (4) providing locations of the connector interfaces such that connecting transmission lines do not obstruct (*e.g*., dangle in front of) the user interface control areas of the overlay panel; and (5) if applicable, providing a height of a connector interface considering its function, such as insufflation gas pressure). Those of ordinary skill in the art would appreciate that other considerations than those listed above may also be taken into account to with regard to the features, arrangements, and positioning of a user interface overlay panel and auxiliary function units to provide an optimized layout and user experience for an auxiliary function control apparatus.

It will also be understood by those of ordinary skill in the art that a complete computer-assisted surgical system, which utilizes an auxiliary function control apparatus in accordance with embodiments of the present disclosure, can have various additional component parts, such as, for example, a manipulator system to which surgical instruments are configured to be mounted for use and a user control system (*e.g*., a surgeon console) for receiving input from a user to the control instruments mounted to the manipulator system and other auxiliary function equipment and medical devices (see FIG. 17 for example), which those having ordinary skill in the art are familiar.

This description and the accompanying drawings that illustrate exemplary embodiments should not be taken as limiting. Various mechanical, compositional, structural, electrical, and operational changes may be made without departing from the scope of the invention as claimed, including equivalents. In some instances, well-known structures and techniques have not been shown or described in detail so as not to obscure the disclosure. Like numbers in two or more figures represent the same or similar elements. Furthermore, elements and their associated features that are described in detail with reference to one embodiment may, whenever practical, be included in other embodiments in which they are not specifically shown or described. For example, if an element is described in detail with reference to one embodiment and is not described with reference to a second embodiment, the element may nevertheless be claimed as included in the second embodiment.

For the purposes of this specification and appended claims, unless otherwise indicated, all numbers expressing quantities, percentages, or proportions, and other numerical values used in the specification and claims, are to be understood as being modified in all instances by the term "about," to the extent they are not already so modified. Accordingly, unless indicated to the contrary, the numerical parameters set forth in the following specification and attached claims are approximations that may vary depending upon the desired properties sought to be obtained. At the very least, and not as an attempt to limit the application of the doctrine of equivalents to the scope of the claims, each numerical parameter should at least be construed in light of the number of reported significant digits and by applying ordinary rounding techniques.

Further, this description's terminology is not intended to limit the invention. For example, spatially relative terms-such as "beneath", "below", "lower", "above", "upper", "proximal", "distal", and the like-may be used to describe one element's or feature's relationship to another element or feature as illustrated in the figures. These spatially relative terms are intended to encompass different positions (i.e., locations) and orientations (i.e., rotational placements) of a device in use or operation in addition to the position and orientation shown in the figures. For example, if a device in the figures is turned over, elements described as "below" or "beneath" other elements or features would then be "above" or "over" the other elements or features. Thus, the exemplary term "below" can encompass both positions and orientations of above and below. A device may be otherwise oriented (rotated 90 degrees or at other orientations) and the spatially relative descriptors used herein interpreted accordingly.

Further modifications and alternative embodiments will be apparent to those of ordinary skill in the art in view of the disclosure herein. For example, the devices may include additional components or steps that were omitted from the diagrams and description for clarity of operation. Accordingly, this description is to be construed as illustrative only and is for the purpose of teaching those skilled in the art the general manner of carrying out the present teachings. It is to be understood that the various embodiments shown and described herein are to be taken as exemplary. Elements and materials, and arrangements of those elements and materials, may be substituted for those illustrated and described herein, parts and processes may be reversed, and certain features of the present teachings may be utilized independently, all as would be apparent to one skilled in the art after having the benefit of the description herein. Changes may be made in the elements described herein without departing from the scope of the following claims.

It is to be understood that the particular examples and embodiments set forth herein are non-limiting; modifications to structure, dimensions, materials, and methodologies may be made without departing from the scope of the present claims.

Other embodiments in accordance with the present invention, defined by the appended claims, will be apparent to those skilled in the art from consideration of the specification and practice of the invention disclosed herein. It is intended that the specification and examples be considered as exemplary only, with the following claims being entitled to their fullest breadth, including equivalents, under the applicable law.

## Claims

1. An apparatus comprising:
a cart (1350, 1450);
a first auxiliary function unit (1302, 1402) removably housed by the cart, the first auxiliary function unit (1302, 1402) comprising one or more first connector interfaces (1408), the one or more first connector interfaces configured to be operably connected to one or more medical devices via mating engagement with one or more first transmission lines;
a second auxiliary function unit (1304, 1404) removably housed by the cart, the second auxiliary function unit (1304, 1404) comprising one or more second connector interfaces (1410), the one or more second connector interfaces configured to be operably connected to one or more medical devices via mating engagement with one or more second transmission lines; and
a user interface overlay panel (1306) coupled to the cart and overlying at least a portion of each of the first auxiliary function unit (1302, 1402) and the second auxiliary function unit (1304, 1404), wherein:
the user interface overlay panel (1306, 1406, 1806) comprises:
one or more first port openings (1828) overlaying the one or more first connector interfaces and configured to receive one or more first transmission line connectors of the one or more first transmission lines, one or more second port openings (1830, 1831) overlaying the one or more second connector interfaces and configured to receive one or more second transmission line connectors, and
a user interface control portion (1850) operably coupled to each of the first auxiliary function unit (1302, 1402) and second auxiliary function unit to alter control settings of each of the first auxiliary function unit (1302, 1402) and second auxiliary function unit (1304, 1404) in response to input at the user interface control portion, wherein the user interface control portion comprises:
a first user interface control area (1812) aligned with the one or more first connector interfaces, the first user interface control area configured to receive input to control the first auxiliary function unit (1302, 1402), and
a second user interface control area (1814) aligned with the one or more second connector interfaces, the second user interface control area configured to receive input to control the second auxiliary function unit (1304, 1404).

2. The apparatus of claim 1, wherein:
the first auxiliary function unit and the second auxiliary function unit are disposed in a stacked arrangement,
the first user interface control area is disposed to overlay at least a portion of the first auxiliary function unit, and
the second user interface control area is disposed to overlay at least a portion of the second auxiliary function unit.

3. The apparatus of claim 1, wherein the user interface control portion comprises a graphical user interface display.

4. The apparatus of claim 1, wherein:
the first auxiliary function unit is configured to control a first auxiliary function;
the second auxiliary function unit is configured to control a second auxiliary function; and
the first auxiliary function and the second auxiliary function differ from each other and are chosen from electrosurgical energy delivery, fluid flow, and imaging.

5. The apparatus of claim 1, wherein the cart further comprises one or more shelves holding the first auxiliary function unit and the second auxiliary function unit in a stacked arrangement.

6. The apparatus of claim 5, wherein the first and second auxiliary function units are removable from the one or more shelves through openings on a side of the one or more shelves opposite a side of the one or more shelves covered by the user interface overlay panel.

7. The apparatus of claim 1, wherein:
the first auxiliary function unit is a surgical insufflation and evacuation unit positioned over the second auxiliary function unit in a stacked arrangement, and
the one or more first connector interfaces comprises a connector interface configured to receive an insufflator hose.

8. The apparatus of claim 7, wherein the second auxiliary function unit is chosen from an electrosurgical unit and an imaging unit.

9. The apparatus of claim 7, further comprising a third auxiliary function unit in the stacked arrangement with the first and second auxiliary function units, wherein:
the third auxiliary function unit comprises one or more third connector interfaces configured to be operably connected to one or more medical devices via mating engagement with one or more third transmission lines;
the user interface overlay panel overlays at least a portion of the third auxiliary function unit;
the user interface control portion comprises a third user interface control area arranged to align with the one or more third connector interfaces; and
the third auxiliary function unit is controllable in response to input at the third user interface control area.

10. The apparatus of claim 9, wherein:
the second auxiliary function unit is an electrosurgical control unit, and
the third auxiliary function unit is an imaging control unit.

11. The apparatus of claim 1, wherein:
the first user interface control area comprises at least one of a first control settings graphical user interface to provide control of the first auxiliary function unit and a first feedback graphical user interface to provide status information about the first auxiliary function unit, and
the second user interface control area comprises at least one of second control settings graphical user interface to provide control of the second auxiliary function unit and a second feedback graphical user interface to provide status information about the second auxiliary function unit.

## Patentansprüche

1. Gerät, umfassend:
einen Wagen (1350, 1450);
eine erste Hilfsfunktionseinheit (1302, 1402), die entfernbar durch den Wagen untergebracht ist, wobei die erste Hilfsfunktionseinheit (1302, 1402) eine oder mehrere erste Steckerschnittstellen (1408) umfasst, wobei die eine oder die mehreren ersten Steckerschnittstellen dazu konfiguriert sind, mittels Passeingriff mit einer oder mehreren ersten Übertragungsleitungen betriebsfähig mit einer oder mehreren medizinischen Vorrichtungen verbunden zu werden;
eine zweite Hilfsfunktionseinheit (1304, 1404), die entfernbar durch den Wagen untergebracht ist, wobei die zweite Hilfsfunktionseinheit (1304, 1404) eine oder mehrere zweite Steckerschnittstellen (1410) umfasst, wobei die eine oder die mehreren zweiten Steckerschnittstellen dazu konfiguriert sind, mittels Passeingriff mit einer oder mehreren zweiten Übertragungsleitungen betriebsfähig mit einer oder mehreren medizinischen Vorrichtungen verbunden zu werden; und
ein Benutzerschnittstellen-Overlay-Bedienfeld (1306), das an den Wagen gekoppelt ist und auf mindestens einem Teil jeder von der ersten Hilfsfunktionseinheit (1302, 1402) und der zweiten Hilfsfunktionseinheit (1304, 1404) aufliegt, wobei:
das Benutzerschnittstellen-Overlay-Bedienfeld (1306, 1406, 1806) umfasst:
eine oder mehrere erste Anschlussöffnungen (1828), die auf der einen oder den mehreren ersten Steckerschnittstellen aufliegen und dazu konfiguriert sind, eine oder mehrere erste Übertragungsleitungsstecker der einen oder der mehreren ersten Übertragungsleitungen aufzunehmen,
eine oder mehrere zweite Anschlussöffnungen (1830, 1831), die auf der einen oder den mehreren zweiten Steckerschnittstellen aufliegen und dazu konfiguriert sind, eine oder mehrere zweite Übertragungsleitungsstecker aufzunehmen, und
einen Benutzerschnittstellensteuerteil (1850), der betriebsfähig an jede von der ersten Hilfsfunktionseinheit (1302, 1402) und der zweiten Hilfsfunktionseinheit gekoppelt ist, um Steuereinstellungen jeder von der ersten Hilfsfunktionseinheit (1302, 1402) und der zweiten Hilfsfunktionseinheit (1304, 1404) als Reaktion auf eine Eingabe an dem Benutzerschnittstellensteuerteil zu verändern, wobei der Benutzerschnittstellensteuerteil umfasst:
einen ersten Benutzerschnittstellensteuerbereich (1812), der auf die eine oder die mehreren ersten Steckerschnittstellen ausgerichtet ist, wobei der erste Benutzerschnittstellensteuerbereich dazu konfiguriert ist, eine Eingabe zu empfangen, um die erste Hilfsfunktionseinheit (1302, 1402) zu steuern, und
einen zweiten Benutzerschnittstellensteuerbereich (1814), der auf die eine oder die mehreren zweiten Steckerschnittstellen ausgerichtet ist, wobei der zweite Benutzerschnittstellensteuerbereich dazu konfiguriert ist, eine Eingabe zu empfangen, um die zweite Hilfsfunktionseinheit (1304, 1404) zu steuern.

2. Gerät nach Anspruch 1, wobei:
die erste Hilfsfunktionseinheit und die zweite Hilfsfunktionseinheit in einer Stapelanordnung angeordnet sind,
der erste Benutzerschnittstellensteuerbereich angeordnet ist, um auf mindestens einem Teil der ersten Hilfsfunktionseinheit aufzuliegen, und
der zweite Benutzerschnittstellensteuerbereich angeordnet ist, um auf mindestens einem Teil der zweiten Hilfsfunktionseinheit aufzuliegen.

3. Gerät nach Anspruch 1, wobei der Benutzerschnittstellensteuerteil eine grafische Benutzerschnittstellenanzeige umfasst.

4. Gerät nach Anspruch 1, wobei:
die erste Hilfsfunktionseinheit dazu konfiguriert ist, eine erste Hilfsfunktion zu steuern;
die zweite Hilfsfunktionseinheit dazu konfiguriert ist, eine zweite Hilfsfunktion zu steuern; und
sich die erste Hilfsfunktion und die zweite Hilfsfunktion voneinander unterscheiden und aus einer Abgabe von elektrochirurgischer Energie, einem Fluidfluss und einer Bildgebung ausgewählt sind.

5. Gerät nach Anspruch 1, wobei der Wagen ferner ein oder mehrere Ablagen umfasst, die die erste Hilfsfunktionseinheit und die zweite Hilfsfunktionseinheit in einer Stapelanordnung halten.

6. Gerät nach Anspruch 5, wobei die erste und die zweite Hilfsfunktionseinheit von der einen oder den mehreren Ablagen durch Öffnungen auf einer Seite der einen oder der mehreren Ablagen entgegengesetzt zu einer Seite der einen oder der mehreren Ablagen, die durch das Benutzerschnittstellen-Overlay-Bedienfeld abgedeckt ist, entfernbar sind.

7. Gerät nach Anspruch 1, wobei:
die erste Hilfsfunktionseinheit eine chirurgische Insufflations- und Entleerungseinheit ist, die über der zweiten Hilfsfunktionseinheit in einer Stapelanordnung positioniert ist, und
die eine oder die mehreren ersten Steckerschnittstellen eine Steckerschnittstelle umfassen, die dazu konfiguriert ist, einen Insufflatorschlauch aufzunehmen.

8. Gerät nach Anspruch 7, wobei die zweite Hilfsfunktionseinheit aus einer elektrochirurgischen Einheit und einer Bildgebungseinheit ausgewählt ist.

9. Gerät nach Anspruch 7, ferner umfassend eine dritte Hilfsfunktionseinheit in der Stapelanordnung mit der ersten und der zweiten Hilfsfunktionseinheit, wobei:
die dritte Hilfsfunktionseinheit eine oder mehrere dritte Steckerschnittstellen umfasst, die dazu konfiguriert sind, mittels Passeingriff mit einer oder mehreren dritten Übertragungsleitungen betriebsfähig mit einer oder mehreren medizinischen Vorrichtungen verbunden zu werden;
das Benutzerschnittstellen-Overlay-Bedienfeld auf mindestens einem Teil der dritten Hilfsfunktionseinheit aufliegt;
der Benutzerschnittstellensteuerteil einen dritten Benutzerschnittstellensteuerbereich umfasst, der dazu eingerichtet ist, sich auf die eine oder die mehreren dritten Steckerschnittstellen auszurichten; und
die dritte Hilfsfunktionseinheit als Reaktion auf eine Eingabe an dem dritten Benutzerschnittstellensteuerbereich steuerbar ist.

10. Gerät nach Anspruch 9, wobei:
die zweite Hilfsfunktionseinheit eine elektrochirurgische Steuereinheit ist und
die dritte Hilfsfunktionseinheit eine Bildgebungssteuereinheit ist.

11. Gerät nach Anspruch 1, wobei:
der erste Benutzerschnittstellensteuerbereich mindestens eine von einer ersten grafischen Benutzerschnittstelle für Steuereinstellungen, um eine Steuerung der ersten Hilfsfunktionseinheit bereitzustellen, und eine erste grafische Benutzerschnittstelle für Rückmeldungen, um Statusinformationen zu der ersten Hilfsfunktionseinheit bereitzustellen, umfasst, und
der zweite Benutzerschnittstellensteuerbereich mindestens eine von einer zweiten grafischen Benutzerschnittstelle für Steuereinstellungen, um eine Steuerung der zweiten Hilfsfunktionseinheit bereitzustellen, und eine zweite grafische Benutzerschnittstelle für Rückmeldungen, um Statusinformationen zu der zweiten Hilfsfunktionseinheit bereitzustellen, umfasst.

## Revendications

1. Appareil comprenant :
un chariot (1350, 1450) ;
une première unité de fonction auxiliaire (1302, 1402) logée de manière amovible dans le chariot, la première unité de fonction auxiliaire (1302, 1402) comprenant une ou plusieurs premières interfaces de connexion (1408), les une ou plusieurs premières interfaces de connexion étant configurées pour être fonctionnellement connectées à un ou plusieurs dispositifs médicaux par accouplement à une ou plusieurs premières lignes de transmission ;
une deuxième unité de fonction auxiliaire (1304, 1404) logée de manière amovible dans le chariot, la deuxième unité de fonction auxiliaire (1304, 1404) comprenant une ou plusieurs deuxièmes interfaces de connexion (1410), les une ou plusieurs deuxièmes interfaces de connexion étant configurées pour être fonctionnellement connectées à un ou plusieurs dispositifs médicaux par accouplement à une ou plusieurs deuxièmes lignes de transmission ; et
un panneau de recouvrement d'interface utilisateur (1306) couplé au chariot et recouvrant au moins une partie de chacune de la première unité de fonction auxiliaire (1302, 1402) et de la deuxième unité de fonction auxiliaire (1304, 1404), dans lequel :
le panneau de recouvrement d'interface utilisateur (1306, 1406, 1806) comprend :
une ou plusieurs premières ouvertures de port (1828) recouvrant les une ou plusieurs premières interfaces de connexion et configurées pour recevoir un ou plusieurs connecteurs de première ligne de transmission des une ou plusieurs premières lignes de transmission,
une ou plusieurs deuxièmes ouvertures de port (1830, 1831) recouvrant les une ou plusieurs deuxièmes interfaces de connexion et configurées pour recevoir un ou plusieurs connecteurs de deuxième ligne de transmission, et
une partie de commande d'interface utilisateur (1850) fonctionnellement couplée à chacune de la première unité de fonction auxiliaire (1302, 1402) et de la deuxième unité de fonction auxiliaire pour modifier des paramètres de commande de chacune de la première unité de fonction auxiliaire (1302, 1402) et de la deuxième unité de fonction auxiliaire (1304, 1404) en réponse à une entrée au niveau de la partie de commande d'interface utilisateur, la partie de commande d'interface utilisateur comprenant :
une première zone de commande d'interface utilisateur (1812) alignée avec les une ou plusieurs premières interfaces de connexion, la première zone de commande d'interface utilisateur étant configurée pour recevoir une entrée pour commander la première unité de fonction auxiliaire (1302, 1402), et
une deuxième zone de commande d'interface utilisateur (1814) alignée avec les une ou plusieurs deuxièmes interfaces de connexion, la deuxième zone de commande d'interface utilisateur étant configurée pour recevoir une entrée pour commander la deuxième unité de fonction auxiliaire (1304, 1404).

2. Appareil selon la revendication 1, dans lequel :
la première unité de fonction auxiliaire et la deuxième unité de fonction auxiliaire sont disposées dans une configuration empilée,
la première zone de commande d'interface utilisateur est disposée de manière à
recouvrir au moins une partie de la première unité de fonction auxiliaire, et la deuxième zone de commande d'interface utilisateur est disposée de manière à
recouvrir au moins une partie de la deuxième unité de fonction auxiliaire.

3. Appareil selon la revendication 1, dans lequel la partie de commande d'interface utilisateur comprend un affichage d'interface utilisateur graphique.

4. Appareil selon la revendication 1, dans lequel :
la première unité de fonction auxiliaire est configurée pour commander une première fonction auxiliaire ;
la deuxième unité de fonction auxiliaire est configurée pour commander une deuxième fonction auxiliaire ; et
la première fonction auxiliaire et la deuxième fonction auxiliaire diffèrent l'une de l'autre et sont choisies parmi l'apport d'énergie électrochirurgicale, l'écoulement d'un fluide, et l'imagerie.

5. Appareil selon la revendication 1, dans lequel le chariot comprend en outre une ou plusieurs étagères portant la première unité de fonction auxiliaire et la deuxième unité de fonction auxiliaire dans une configuration empilée.

6. Appareil selon la revendication 5, dans lequel les première et deuxième unités de fonction auxiliaire peuvent être retirées des une ou plusieurs étagères par des ouvertures sur un côté des une ou plusieurs étagères à l'opposé d'un côté des une ou plusieurs étagères recouvert par le panneau de recouvrement d'interface utilisateur.

7. Appareil selon la revendication 1, dans lequel :
la première unité de fonction auxiliaire est une unité d'insufflation et d'évacuation chirurgicale positionnée au-dessus de la deuxième unité de fonction auxiliaire dans une configuration empilée, et
les une ou plusieurs premières interfaces de connexion comprennent une interface de connexion configurée pour recevoir un tuyau d'insufflation.

8. Appareil selon la revendication 7, dans lequel la deuxième unité de fonction auxiliaire est choisie parmi une unité électrochirurgicale et une unité d'imagerie.

9. Appareil selon la revendication 7, comprenant en outre une troisième unité de fonction auxiliaire dans la configuration empilée avec les première et deuxième unités de fonction auxiliaire, dans lequel :
la troisième unité de fonction auxiliaire comprend une ou plusieurs troisièmes interfaces de connexion configurées pour être fonctionnellement connectées à un ou plusieurs dispositifs médicaux par accouplement à une ou plusieurs troisièmes lignes de transmission ;
le panneau de recouvrement d'interface utilisateur recouvre au moins une partie de la troisième unité de fonction auxiliaire ;
la partie de commande d'interface utilisateur comprend une troisième zone de commande d'interface utilisateur agencée de manière à s'aligner avec les une ou plusieurs troisièmes interfaces de connexion ; et
la troisième unité de fonction auxiliaire peut être commandée en réponse à une entrée au niveau de la troisième zone de commande d'interface utilisateur.

10. Appareil selon la revendication 9, dans lequel :
la deuxième unité de fonction auxiliaire est une unité de commande électrochirurgicale, et
la troisième unité de fonction auxiliaire est une unité de commande d'imagerie.

11. Appareil selon la revendication 1, dans lequel :
la première zone de commande d'interface utilisateur comprend au moins l'une d'une première interface utilisateur graphique pour les paramètres de commande permettant de commander la première unité de fonction auxiliaire, et une première interface utilisateur graphique de rétroaction pour fournir des informations d'état sur la première unité de fonction auxiliaire, et
la deuxième zone de commande d'interface utilisateur comprend au moins l'une d'une deuxième interface utilisateur graphique pour les paramètres de commande permettant de commander la deuxième unité de fonction auxiliaire, et une deuxième interface utilisateur graphique de rétroaction pour fournir des informations d'état sur la deuxième unité de fonction auxiliaire.
